# EUROPÄISCHE PATENTSCHRIFT

(11) **EP 1 229 803 B1**
(45) Veröffentlichungstag und Bekanntmachung des Hinweises auf die Patenterteilung: **10.05.2006**
(21) Anmeldenummer: 00993030.6
(22) Anmeldetag: 10.11.2000
(51) Int. Cl.: A23L 1/00

(54) **VERWENDUNG EINES MODIFIZIERTEN KOHLENHYDRATS**
USE OF A MODIFIED CARBOHYDRATE
UTILISATION D'UN HYDRATE DE CARBONE MODIFIÉ

(30) Priorität: 11.11.1999 DE 19954233
(43) Veröffentlichungstag der Anmeldung: 14.08.2002
(73) Patentinhaber: N.V. Nutricia, 2712 HM Zoetermeer (NL)
(72) Erfinder: STAHL, Bernd, 61381 Friedrichsdorf (DE); KLIEM, Michael, 91086 Aurachtal-Falkendorf (DE); FARWER, Sandra, 41564 Kaarst (DE); SAWATZKI, Günther, 35516 Münzenberg (DE); BOEHM, Günther, 61209 Echzell (DE)
(74) Vertreter: Köster, Hajo
(86) Internationale Anmeldenummer: PCT/EP2000/011134
(87) Internationale Veröffentlichungsnummer: WO 2001/033973

(56) Entgegenhaltungen:
- WO-A-00/08948
- US-A- 4 219 571
- FU D ET AL: "MALTODEXTRIN ACCEPTOR REACTIONS OF STREPTOCOCCUS-MUTANS 6715 GLUCOSYLTRANSFERASES" CARBOHYDRATE RESEARCH, Bd. 217, 1991, Seiten 201-212, XP002166087 ISSN: 0008-6215
- FU D ET AL: "ACCEPTOR REACTIONS OF MALTODEXTRINS WITH LEUCONOSTOC-MESENTEROIDES B-512FM DEXTRANSUCRASE" ARCHIVES OF BIOCHEMISTRY AND BIOPHYSICS, Bd. 283, Nr. 2, 1990, Seiten 379-387, XP000992861 ISSN: 0003-9861
- QU-MING GU: "Enzyme-mediated reactions of oligosaccharides and polysaccharides" J ENVIRON POLYM DEGRAD;JOURNAL OF ENVIRONMENTAL POLYMER DEGRADATION 1999 KLUWER ACADEMIC/PLENUM PUBL CORP, NEW YORK, NY, USA, Bd. 7, Nr. 1, 1999, Seiten 1-7, XP000992950
- RENDLEMAN J A JR: "Enhanced production of.Nd-cyclodextrin from corn syrup solids by means of cyclododecanone as selective complexant." CARBOHYDRATE RESEARCH 1993 BIOPOLYMER RES. UNIT, NAT. CENT. FOR AGRIC. UTIL. RES., USDA, ARS, PEORIA, IL 61604, USA, Bd. 247, Seiten 223-237, XP000394390

## Beschreibung

Die Erfindung betrifft die Verwendung eines modifizierten Kohlenhydrats a) oder b), das durch enzymatische Kopplung bzw. Verknüpfung eines üblichen, zu Ernährungszwecken eingesetzten Kohlenhydrates mit einem weiteren Kohlenhydratrest erhalten wurde, oder einer Mischung aus mehreren derartigen Kohlenhydraten zur Herstellung eines Mittels in Form eines Diabetikernahrungsmittels oder eines Pharmazeutikums für einen Diabetiker,

Für Diabetiker ist die Zuführung glucosehaltiger Kohlenhydrate kritisch, da durch den Insulinmangel die nach der Verdauung dieser Nahrungsmittel und der anschließenden Aufnahme der Glucose in die Blutbahn die in den Körper gelangte Glucose quantitativ nicht ausreichend verwendet werden kann. Die Folge davon ist eine hohe Glucosekonzentration im Plasma, die ihrerseits pathologische metabolische Reaktionen hervorruft.

In der üblichen menschlichen Ernährung spielen glucosehaltige Nahrungsmittel,eine große Rolle. Die wichtigsten Glucosequellen in der menschlichen Ernährung sind Glucosepolymere aus pflanzlichen Nahrungsmitteln. Die Glucosemoleküle sind dabei in sogenannter α 1→4-glycosidischer Bindung linear verknüpft (Amylose). Durch sogenannte α 1→6-glycosidische Verzweigungen entstehen aus Amylosen die Amylopektine. Jeder Zweig besteht jedoch wiederum aus Glucosen in α 1→4-glycosidischer Bindung. Das entsprechend verzweigte Glucosepolymer aus tierischen Nahrungsmitteln wird Glycogen genannt. Sowohl die α 1→4-glycosidische Bindung als auch die α 1→6-glycosidischen Verzweigungsstellen können schnell durch körpereigene Hydrolasen, beispielsweise Amylasen, Amyloglucosidasen und Maltasen, in Maltodextrine, Maltose und schließlich Glucose gespalten und abgebaut werden.

Bei der Verabreichung der üblichen glucosehaltigen Nahrungen wird die Glucose sehr rasch in die Blutbahn aufgenommen, so daß schon 15 min danach und somit postprandial hohe Glucosekonzentrationen beobachtet werden können, die bei stoffwechselgesunden Menschen jedoch rasch wieder abfallen. Dieser rasche Abfall der Glucosekonzentration im Plasma wird durch die schnelle Aufnahme der Glucose in den Stoffwechsel der Zellen bedingt.

Beim Diabetiker ist dieser Einstrom der Glucose in die Zellen gestört, so daß der geschilderte Abfall, in Abhängigkeit vom Schweregrad des Diabetes Mellitus, verzögert abläuft, und somit hohe Glucosekonzentrationen im Plasma persistieren.

Glucosehaltige Nahrungsmittel sind allerdings auch für Diabetiker unverzichtbar, da Glucose eine Grundsubstanz für den Energiestoffwechsel aller Zellen darstellt. Außerdem sind glucosehaltige Kohlenhydrate traditionell wichtige Bestandteile der menschlichen Ernährung. Der Verzicht auf glucosehaltige Kohlenhydrate wird somit - neben der metabolischen Bedeutung eines Glucosemangels - auch erhebliche Einschränkungen der Lebensqualität für den Diabetiker mit sich bringen.

Für Diabetiker wäre es nun wünschenswert, wenn die Nahrungsaufnahme nicht zu den geschilderten hohen Schwankungen der Glucosekonzentration im Plasma führen würde. Um dies zu erreichen, sind bereits verschiedene Konzepte entwickelt worden. Zum einen versucht man eine Verstetigung der Blutglucose-Konzentration dadurch zu erreichen, daß der Kohlenhydratanteil in Nahrungen zugunsten des Fettanteiles reduziert wird. Zu diesem Zweck bietet beispielsweise die Firma Abbott die Nahrung Glucerna® an. Der prozentuale Anteil der Nahrungsenergie in dieser bekannten Nahrung ist wie folgt: 50,7 % der Energie aus Fett, 16,9 % der Energie aus Proteinen und 32,4 % der Energie aus Kohlenhydraten

Es ist auch schon versucht worden, die Blutglucoseschwankungen durch Verabreichung von Nahrungen zu reduzieren, die als Kohlenhydrate eine Mischung von gut absorbierbaren und schlecht absorbierbaren Kohlenhydraten enthalten. Diesbezüglich wird verwiesen auf EP 0 768 043 bzw. WO 96131129.

Ferner istes beispielsweise aus "Carbohydrate Research, 217 (1991) 201-211" bekannt, dass Maltodertrine als für übertragene Glucose dienen Können. In "Arch, Biochem. Biophys. 283, 379-387,1990 werden kreptorreaktionen von Maltodextrinen bei der Verwendung von Dextran sucrase beschrieben.

In der nicht vor veroffentlichten WO-A-0 008 948 sind Kohlenhydratmischungen beschrieben, die zwei Komponenten unterschied Lange und Struktur enthalten und prabiotisch wirken.

Aufgabe der vov liegen den Erfindung ist es, modifizierte Kohlenhydrate aufzuzeigen, die in Diabetikernahrungsmitteln und in Pharmazeutika für Diabetiker einge setzt werden können.

Gelöst wird diese Aufgabe durch die Lehre der Ansprüche.

Zur Herstellung der erfindungsgemäß modifizierten Kohlenhydrate dienen übliche, zu Ernährungszwecken eingesetzte Kohlenhydrate. Dabei finden zwei Gruppen von Kohlenhydraten Anwendung. Untereinem erfindungsgemäßen Kohlen hydrat wird hierein erfindungsgemäß verwendetes Kohlen hydrat verstanden.

Bei der ersten Gruppe handelt es sich um Kohlenhydrate mit einem verdaubaren, glucosehaltigen Grundgerüst. An diesen Grundkörper wird mindestens ein weiterer Glucoserest oder ein anderer Kohlenhydratrest gekoppelt und somit damit verbunden. Bei diesem anderen angekoppelten Kohlenhydratrest kann es sich um ein Monosaccharid, Disaccharid, Oligosaccharid oder Polysaccharid handeln. Dieser andere Kohlenhydratrest kann zudem weitere Glucoseeinheiten aufweisen oder auch frei von derartigen Glucoseeinheiten sein. Derartig erfindungsgemäß modifizierte Kohlenhydrate mit einem verdaubaren Grundkörper (bzw. Core) und einem daran angekoppelten weiteren Glucoserest und/oder Kohlenhydratrest werden im Rahmen der hier vorliegenden Unterlagen als Kohlenhydrate a) bezeichnet.

Durch Anfügen bzw. Ankoppeln eines weiteren Glucoserestes und/oder eines anderen Kohlenhydratrestes an diese üblichen verdaubaren glucosehaltigen Grundkörper werden sogenannte "slow release-Kohlenhydrate" erhalten, deren Abbaubarkeit bzw. Verdaubarkeit im Magen- bzw. Darmtrakt durch Ankoppeln der genannten Reste erschwert wird.

Die zweite Gruppe an erfindungsgemäß einsetzbaren und modifizierten Kohlenhydrate besitzen als Grundkörper ein nicht verdaubares Kohlenhydrat, an das mindestens ein Glucoserest und/oder ein glucosehaltiger Kohlenhydratrest gekoppelt und somit damit verbunden wird. Diese modifizierten Kohlenhydrate werden im Rahmen der vorliegenden Unterlagen als Kohlenhydrate b) bezeichnet.

Derartige Grundkörper aus nicht-verdaubaren Kohlenhydraten sind beispielsweise Dextrane und Fructane und Cellulosen. Diese dienen als Träger von damit verbunden bzw. daran angekoppelten Glucoseresten und/oder glucosehaltigen Kohlenhydratresten bzw. Glucoseoligomeren. Bei den angekoppelten Kohlenhydratresten kann es sich dabei um die gleichen handeln wie bei den Kohlenhydraten a). Allerdings müssen die angekoppelten Kohlenhydratreste mindestens eine Glucoseeinheit enthalten. Diese angekoppelten glucosehaltigen Kohlenhydratreste können von dem damit verbundenen Träger nur langsam wieder freigesetzt werden. Die Menge an maximal freisetzbarer Glucose entspricht dabei derjenigen Glucosemenge, die zuvor an den Grundkörper angekoppelt wurde.

Die erfindungsgemäß modifizierten Kohlenhydrate a) und b) können durch körpereigene Glycosidasen (beispielsweise Hydrolasen im Speichel, Pankreassaft, Dünndarm-Disaccharidasen) erschwert abgebaut werden (betrifft Kohlehydrate a)) oder trotz unverdaübarem Grundkörpers überhaupt abgebaut werden und stellen somit "slow-release-Kohlenhydrate" dar.

Die erfindungsgemäß modifizierten Kohlenhydrate werden dabei vorzugsweise durch enzymatische Ankopplung der damit verbundenen Kohlenhydratreste erhalten.

Die erfindungsgemäß modifizierten Kohlenhydrate a) und b) weisen einen Polymerisationsgrad '(degree of polymerization, DP) von mindestens 2 (d.h. Disaccharide) bis zu einigen 100.000 auf. Bevorzugt ist ein Bereich von DP 3 bis 100.

Die erfindungsgemäß modifizierten Kohlenhydrate a) und b) bestehen somit - wie oben dargelegt - aus einem von vorne herein verdaubaren Grundkörper oder einem von vorneherein nicht verdaubaren Grundkörper, wobei dieser Grundkörper mit mindestens einem weiteren Kohlenhydratrest gekoppelt ist, insbesondere durch enzymatische Verknüpfung. Sowohl der Grundkörper als auch der angekoppelte Kohlenhydratrest bestehen im einfachsten Fall aus jeweils mindestens einem Monosaccharid. Das Verhältnis zwischen der Anzahl der Monosaccharideinheiten im Grundkörper und der Anzahl der Monosaccharideinheiten in der bzw. den mit dem Grundkörper verknüpften Gruppe(n) beträgt vorzugsweise jedoch 1:10 bis 100:1.

Besteht ein Grundkörper aus mindestens drei Monosaccharideinheiten, dann führt die Verknüpfung mit einem weiteren Monosaccharid entweder zu einer Verlängerung des Grundkörpers oder zu einer Verzweigung des Grundkörpers. Besteht der Grundkörper aus mehr als drei Monosacchariden, dann können mehrere Verzweigungen eingeführt werden. Derartige Verzweigungen führen im erfindungsgemäßen Sinne zu einer zusätzlichen sterischen Behinderung körpereigener Glycosidasen. Durch derartige Verzweigungen können gezielt "slow-release-Kohlenhydrate" erhalten werden.

Bei dem verdaubaren Grundkörper bzw. Kohlenhydrat des modifizierten Kohlenhydrates a) handelt es sich um Maltodextrine.

Bei dem verdaubaren Grundkörper bzw. Kohlenhydrat des modifizierten Kohlenhydrates b) handelt es sich vorzugsweise um Fructane, β-Glucane, Cellulose, Pektine, Galacturonane, Galactane, Galactomannane, β-Galacto-Oligosaccharide, α-Galacto-Oligosaccharide, Fucoidane, Mannane, Xylane, Xyloglucane, Laminarin, Chitine, Chitosane, Hyaluronsäuren, Chondroitine, Proteoglycane, Glucurono-Oligosaccharide, Arabinane, Arabinoxylane, Arabinogalactane, Rhamno-Oligosaccharide, Xanthane, Alginate, Agar, Carageenane, Hemicellulosen, Pflanzengummi, enzymatisch hergestellte Kohlenhydrate (z.B. Galacto-Oligosaccharide und Gluco-Oligosaccharide), bakterielle Kohlenhydrate (beispielsweise Xanthane, Dextrane und Sialyl-Oligosaccharide), N-Glycoprotein-Oligosaccharide, O-Glycoprotein-Oligosaccharide und Glycolipid-Oligosaccharide.

Die erfindungsgemäße Kohlenhydratmischung kann somit im einfachsten Fall aus einem oder mehreren erfindungsgemäß modifizierten Kohlenhydrat(en) a) bestehen.

Die erfindungsgemäße Kohlenhydratmischung kann ferner aus einem oder mehreren erfindungsgemäß modifizierten Koh(enhydrat(en) b) bestehen.

Die erfindungsgemäße Kohlenhydratmischung kann ferner aus einer Mischung aus einem oder mehreren erfindungsgemäß modifizierten Kohlenhydrat(en) a) mit einem oder mehreren erfindungsgemäß modifizierten Kohlenhydrat(en) b) bestehen.

Weiterhin können in der erfindungsgemäßen Kohlenhydratmischung neben einem oder mehreren erfindungsgemäß modifizierten Kohlenhydrat(en) a) und/oder neben einem oder mehreren erfindungsgemäß modifizierten Kohlenhydrat(en) b) auch übliche, für die Herstellung von Nahrungen eingesetzte, nicht modifizierte Kohlenhydrate c) vorhanden sein, die somit nicht im erfindungsgemäßen Sinne modifiziert wurden. Bei der Beurteilung, ob bei einer Kohlenhydratmischung, die darin vorhandenen Kohlenhydrate als Kohlenhydrat a), als Kohlenhydrat b) oder Kohlenhydrat c) anzusehen sind, ist zu beachten, daß zu den Kohlenhydraten c) nicht nur solche zählen, die von Anfang an unmodifiziert bei der Herstellung der Kohlenhydratmischung bzw. einer damit versehenen Nahrung zugegeben wurden. Vielmehr zählen zu den Kohlenhydraten c) auch solche Kohlenhydrate, die bei dem Herstellungsverfahren erfindungsgemäß modifizierter Kohlenhydrate nicht reagiert haben und somit nicht modifiziert und daher auch nicht verändert wurden. Mit anderen Worten bedeutet dies folgendes. Werden bei der erfindungsgemäßen Modifizierung einige der eingesetzten Grundkörper mit keinem weiteren Kohlenhydratrest gekoppelt und bleiben sie somit unverändert, dann sind sie den Kohlenhydraten c) zuzurechnen. Gleiches gilt für die Kohlenhydratreste, die mit den Grundkörpern, sei er nun verdaubar oder unverdaubar, gekoppelt werden sollen. Auch diejenigen Kohlenhydratreste, die nicht reagiert haben und somit nicht an einen Grundkörper gekoppelt wurden, zählen zu den Kohlenhydraten c).

Dabei ist von Bedeutung, daß die zur Anwendung gebrachte Mischung von Kohlenhydraten derart zusammengesetzt ist, daß in einem in-vitro Verdausystem auf Pankreatinbasis mindestens 10 Gew.-% weniger Glucose pro Zeiteinheit freigesetzt wird, verglichen mit einer Kohlenhydratmischung, welche die gleiche Gewichtsmenge an nicht modifiziertem Kohlenhydrat oder an nicht modifizierten Kohlenhydraten und/oder an den zur Herstellung des (der) modifizierten Kohlenhydrate(s) a) und b) eingesetzten Ausgangskohlenhydraten enthält. Mit anderen Worten bedeutet dies folgendes. Die Freisetzung von Glucose aus der erfindungsgemäßen Kohlenhydratmischung ist um mindestens 10 % reduziert gegenüber der Freisetzung von Glucose aus einer Kohlenhydratmischung, welche die gleiche Gewichtsmenge an Kohlenhydraten enthält, wobei diese Kohlenhydrate zusammengesetzt sind aus der Summe an nicht modifizierten Kohlenhydraten, sofern diese vorhanden sind, und den Ausgangskohlenhydraten, die zur Herstellung der modifizierten Kohlenhydrate a) und b) eingesetzt wurden. Diese 10 %-ige Reduktion bezieht sich auf einen Zeitraum vom Start der Verdauung bis zu 90 min nach Verdauungsbeginn.

Um letzteres zu erläutern, wird einmal angenommen, daß ein erfindungsgemäßes Kohlenhydrat ausschließlich aus einem erfindungsgemäß modifizierten Kohlenhydrat a) besteht. Zur Herstellung dieses erfindungsgemäßen Kohlenhydrates a) werden 5 g eines Glucoserestes oder eines anderen Kohlenhydratrestes mit 15 g eines Kohlenhydrates mit einem verdaubaren glucosehaltigen Grundkörper verknüpft. Dabei werden 20 g erfindungsgemäß modifiziertes Kohlenhydrat a) erhalten.

Die Verdaubarkeit dieses erfindungsgemäß modifizierten Kohlenhydrates a) muß nun derart verändert sein, daß in einem in-vitro Verdausystem auf Pankreatinbasis mindestens 10 Gew.-% weniger Glucose pro Zeiteinheit freigesetzt wird, verglichen mit der ursprünglichen und somit der vor der Kopplung eingesetzten Kohlenhydratmischung aus 15 g Grundkörper und 5 g des damit zu koppelnden Glucoserestes und/oder Kohlenhydratrestes.

Analoges gilt für den Fall, daß die erfindungsgemäße Kohlenhydratmischung aus einem oder mehreren erfindungsgemäß modifizierten Kohlenhydrat(en) b) oder einer Mischung aus einem oder mehreren erfindungsgemäß modifizierten Kohlenhydrat(en) a) und einem oder mehreren erfindungsgemäß modifizierten Kohlenhydrat(en) b) besteht. Analoges gilt ferner, wenn neben dem oder den erfindungsgemäß modifizierten Kohlenhydrat(en) a) und/oder dem oder den erfindungsgemäß modifizierten Kohlenhydrat(en) b) auch ein oder mehrere übliche, nicht modifizierte Kohlenhydrate(en) vorhanden ist (sind).

Dabei ist - wie oben dargelegt - zu berücksichtigen, daß bei einer Kopplung der Glucosereste oder der anderen Kohlenhydratreste mit dem beschriebenen Grundkörper nicht unbedingt an jedes Grundkörpermolekül ein Glucoserest oder ein anderer Kohlenhydratrest gekoppelt wird. Auch muß nicht jeder ursprünglich eingesetzte Glucoserest oder anderer Kohlenhydratrest unbedingt eine Kopplungsreaktion mit einem Grundkörper eingehen, sondern kann auch nach Durchführung der Kopplungsreaktion noch in der ursprünglichen Form vorhanden sein. Diese nicht umgesetzten Reste sind dann den Kohlenhydraten c) zuzurechnen.

Entscheidend ist lediglich, daß in einem in-vitro Verdausystem auf Pankreatinbasis mindestens 10 Gew.-% weniger Glucose pro Zeiteinheit freigesetzt wird, verglichen mit einer Mischung der ursprünglich eingesetzten Ausgangskohlenhydrate.

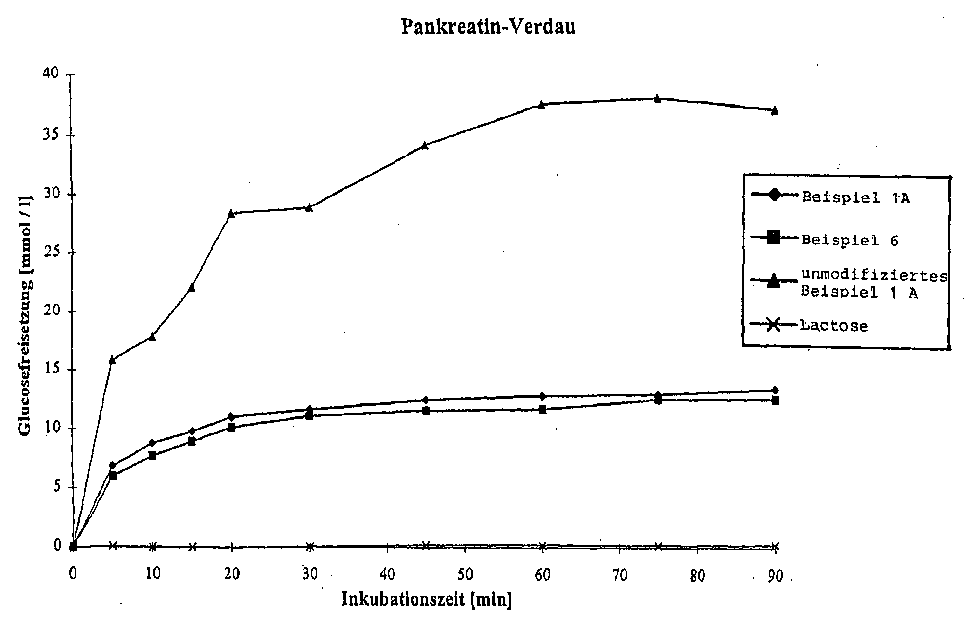

Die hier angesprochene Verdaubarkeit von Kohlenhydraten wird im übrigen in-vitro experimentell bestimmt, indem die erfindungsgemäßen Kohlenhydratmischungen mittels Pankreatin vom Schwein (z.B. Sigma) inkubiert werden. Die eingesetzte Konzentration des Pankreatin in Wasser beträgt dabei 10 g/l mit 1 mM Serinproteasehemmer Pefabloc (Boehringer Mannheim) und 0,02 % NaN₃. 1 ml sterilfiltrierter Pankreatinlösung werden mit 200 µl Lösung der Kohlenhydratmischung (10 mg/ml) und 800 µl Wasser vermischt und bei 37 °C für 90 min inkubiert. Die freigesetzte Glucose wird mittels eines enzymatischen Tests (z.B. Boehringer Testkit, Best.Nr. 139106) bestimmt. Zu den Zeitpunkten 5 min, 10 min, 15 min, 20 min, 30 min, 45 min, 60 min, 75 min und 90 min werden dazu Aliquots entnommen. Die Reaktion wird durch Inkubation bei 95 °C für 5 min abgestoppt. Als Negativkontrolle für den Pankreatinverdau wird Lactose eingesetzt. Als positive Kontrolle dient Maltodextrin. Ein Beispiel für eine derartige Verdauung für zwei erfindungsgemäße slow-release-Kohlenhydratmischungen ist in der Abbildung dargestellt. In dieser Abbildung ist die Glucosefreisetzung für zwei erfindungsgemäße Kohlenhydratmischungen, nämlich die in den Beispielen 1 a und 6 beschriebenen, verglichen mit der Glucosefreisetzung aus einer Kohlenhydratmischung, welche aus den Ausgangskohlenhydraten des Beispieles 1 a, nämlich Saccharose und Maltodextrine, besteht. Der Unterschied zwischen den Kurven für das Beispiel 1 a und für das unmodifizierte Beispiel 1 a besteht somit darin, daß die eingesetzten Kohlenhydrate beim Beispiel 1 a mit Dextransucrase behandelt wurden, während eine derartige Behandlung beim unmodifizierten Beispiel 1 a nicht erfolgte.

Die aus der erfindungsgemäßen Kohlenhydratmischung verzögert freigesetzte Glucose führt unmittelbar nach der Nahrungsaufnahme zu geringeren Peak-Glucosekonzentrationen und auch über die unmittelbare postprandiale Phase hinaus zu relative gleichbleibenden Glucosekonzentrationen im Plasma, wodurch trotz des bestehenden Insulinmangels des Diabetikers die Glucose verstoffwechselt werden kann und somit als wichtiger Energieträger zur Verfügung steht. Die verminderte Verdaubarkeit bedingt außerdem das ein Teil der erfindungsgemäßen Kohlenhydratmischung in den Dickdarm gelangen kann, wo eine weitere Verwertung der erfindungsgemäßen Kohlenhydratmischung durch die bestehende Darmflora erfolgt. Dies hat - neben der weiteren Bereitstellung von Glucose für den Organismus - die positive Wirkung, daß die intestinale Mikroflora stimuliert wird, wodurch Verdauungsprobleme reduziert werden können.

Die eingesetzten, erfindungsgemäß modifizierten Kohlenhydratstrukturen bestehen dabei aus einem Grundkörper bzw. Core und aus damit verknüpften terminalen Gruppen. Die Cores werden hinsichtlich ihrer Verdaubarkeit durch die terminalen Gruppen beeinflußt, wodurch letztendlich eine Veränderung der Kinetik der intraluminalen Verdauung bedingt ist. Diese veränderte Verdaubarkeit beeinflußt die Kinetik der Glucoseaufnahme aus dem Darm in die Blutbahn, so daß der üblicherweise nach 15 min auftretende Glucosepeak im Plasma (Petrides et al. Aus Diabetes mellitus 5. Aufl. (Ed. Petrides, Weiss, Löffler, Wieland) 1985, Urban & Schwarzenberg, München) bei Verabreichung einer erfindungsgemäßen Kohlenhydratmischung oder eine diese Kohlenhydratmischung enthaltenden Nahrung vermieden wird.

Die erfindungsgemäßen Kohlenhydrate werden in einer Diabetikernahrung bzw. einem Diabetikernahrungsmittel eingesetzt. Daneben können weitere Bestandteile vorhanden sein, beispielsweise Fette und Proteine sowie gegebenenfalls weitere, üblicherweise eingesetzte Ingredienzien. Die erfindungsgemäße Kohlenhydratmischung kann zudem als oder zur Herstellung von Sondennahrung eingesetzt werden.

Ein weiteres Einsatzgebiet betrifft den pharmazeutischen Sektor. Somit kann die erfindungsgemäße Kohlenhydratmischung auch in Pharmazeutika Anwendung finden.

Für die Herstellung der erfindungsgemäß modifizierten Kohlenhydrate a) und b) mittels enzymatischer Verknüpfung werden vorzugsweise die folgenden Enzymklassen eingesetzt:
A. Natürliche und/oder rekombinante Glycosidasen aus biologischen Quellen, wie Pflanzen, Tieren und Mikroorganismen, die über eine reverse Hydrolase oder in einer Transglycosylierungs-Reaktion in Richtung Synthese arbeiten. Beispielsweise kann die β-Galactosidase (EC 3.2.1.23) aus *Aspergillus oryzae* eingesetzt werden. Als weitere Quellen für die β-Galactosidase dienen in erster Linie Mikroorganismen oder Einzeller, beispielsweise *Kluiveromyces lactis, Streptococcus thermophilus, Lactobacillus bulgaricus* und *Bacillus circulans..*
   Des weiteren können α-Glucosidasen, beispielsweise Amyloglucosidase aus *Aspergillus niger* (EC 3.2.1.3), α-Amylase, beispielsweise aus *Bacillus amyloliquefaciens* (EC 3.2.1.1) sowie beispielsweise aus *Alcaligenes spec.,* Pullulanasen (EC 3.2.1.41) und/oder β-Glucosidasen, beispielsweise die aus Süßmandeln (EC 3.2.1.21), oder Cellulase, beispielsweise aus *Trichoderma viride* (EC 3.2.1.4) verwendet werden. Auch N-Acetyl-β-D-glucosamidase, beispielsweise aus Rindernieren, kann Anwendung finden.
B. Natürliche und/oder rekombinante Transferasen aus biologischen Quellen wie Pflanzen, Tieren und Mikroorganismen, die keine aktivierten Mono-/Oligosaccharide als Substrat benötigen. Dazu zähen Cyclomaltodextrin-Glucanotransferase CGT (EC 2.3.1.19), beispielsweise aus *Bacillus macerans,* und Dextransucrase (EC 2.4.1.5), beispielsweise aus *Leuconostoc mesenteroides.* Ferner zählt Transglucosidase, beispielsweise aus *Aspergillus niger* (EC 2.4.1.24), dazu.
   und/oder
C. natürliche und/oder rekombinante Transferasen aus biologischen Quellen wie Pflanzen, Tiere und Mikroorganismen, die aktivierte Monosaccharide als Substrat benötigen, beispielsweise die Galactosyl-Transferase aus beispielsweise boviner Milch (EC 2.4.1.22). Dieses Enzym transferiert Galactose von UDP-Galactose als Donor in β 1->4-Bindungen an Glucosereste.

Die folgenden Beispiele dienen zur Erläuterung der Erfindung.

### Beispiel 1

Herstellung eines erfindungsgemäß modifizierten Kohlenhydrates a) aus einem verdaubaren Grundkörper und mindestens einem damit verknüpften Glucoserest.

### Variante A)

20 g Saccharose und 100 g Maltodextrine (gut wasserlösliche niedermolekulare Amylose unterschiedlicher Kettenlänge) in 500 ml 20 mM Acetatpuffer werden bei pH 5,2 mit 2.000 U Dextransucrase aus *Leuconostoc mesenteroides* (EC 2.4.1.24) 5 h bei 37 °C inkubiert. Dabei wird mindestens ein aus der Saccharose stammende Glucoserest auf die Maltodextrineinheiten übertragen. Das so erhaltene modifizierte Maltodextrin wird nach Hitzedenaturierung des Enzyms (5 min bei 100 °C) durch Ultrafiltration aufgereinigt. Es werden 40 g durch mindestens einen Glucoserest modifizierte Maltodextrine erhalten.

### Variante B)

5 g Maltose und 15 g Maltodextrine (gut wasserlösliche niedermolekulare Amylose unterschiedlicher Kettenlänge) in 500 ml 10 mM Acetatpuffer werden bei pH 4,0 mit 10.000 U Transglucosidase aus Aspergillus niger (EC 2.4.1.24) 5 h bei 37 °C inkubiert. Dabei wird mindestens ein aus der Maltose stammende Glucoserest auf die Maltodextrineinheiten übertragen. Das so erhaltene modifzierte Maltodextrin wird nach Hitzedenaturierung des Enzyms (5 min bei 100 °C) durch Ultrafiltration aufgereinigt. Es werden 12,5 g durch mindestens einen Glucoserest modifizierte Maltodextrine erhalten.

### Variante C)

Es wird ebenso verfahren wie bei der Variante A), wobei jedoch anstelle von 37 °C bei 50 °C inkubiert wurde. Es werden 40 g durch mindestens einen Glucoserest modifizierte Maltodextrine erhalten.

### Variante D)

Es wird ebenso verfahren wie bei der Variante B), wobei jedoch anstelle von 37 °C bei 50 °C inkubiert wurde. Es werden 12,5 g durch mindestens einen Glucoserest modifzierte Maltrodextrine erhalten.

### Variante E)

Es wird ebenso verfahren wie bei der Variante A), wobei jedoch anstelle von 37 °C bei 10 °C inkubiert wurde. Es werden 40 g durch mindestens einen Glucoserest modifizierte Maltodextrine erhalten.

### Variante F)

Es wird ebenso verfahren wie bei der Variante B), wobei jedoch anstelle von 37 °C bei 10 °C inkubiert wurde. Es werden 12,5 g durch mindestens einen Glucoserest modifzierte Maltodextrine erhalten.

### Beispiel 2

Herstellung eines erfindungsgemäß modifizierten Kohlenhydrates a) aus einem verdaubaren Grundkörper und mindestens einem daran angekoppelten, glucosehaltigen Kohlenhydratrest.

Auf 30 g Maltodextrine werden Glucanreste aus 10 g Amylase oder Amylopektin (aus Stärke) mittels Cyclomaltodextrin-Glucanotransferase CGT (EC 2.4.1.19) aus *Bacillus macerans* CGT übertragen.

### Beispiel 3

Herstellung eines erfindungsgemäß modifizierten Kohlenhydrates a) aus einem verdaubaren Gundkörper und mindestens einem daran angekoppelten, nicht-glucosehaltigen Kohlenhydratrest.

Übertragung von Galactose auf Maltodextrin.

### Variante A)

100 g Maltodextrin (gut wasserlösliche niedermolekulare Amylose unterschiedlicher Kettenlänge) und 10 g Lactose werden in 500 ml 40 mM NaOAc Puffer pH 5,0 mit 2000 U β-Galactosidase aus *Kluiveromyces lactis* für 5 h bei 37 °C inkubiert. Die so erhaltenen galactosylierten Maltodextrine werden nach Hitzedenaturierung des Enzyms (5 min bei 100 °C) durch Ultratfiltration aufgereinigt.

### Variante B)

Es wird ebenso verfahren wie bei der Variante A), wobei jedoch bei 50 °C statt bei 37 °C inkubiert wird.

### Variante C)

Es wird ebenso verfahren wie bei der Variante A), wobei jedoch bei 10 °C statt bei 37 °C inkubiert wird.

### Beispiel 4

Herstellung eines erfindungsgemäß modifizierten Kohlenhydrates b) aus einem unverdaubaren Grundkörper und mindestens einem daran angekoppelten Glucoserest.

### Variante A)

50 g Cello-Oligosaccharide (DP2 bis DP 10) und 15 g Maltose in 500 ml 20 mM Acetatpuffer werden bei pH 5,2 mit 30000 U Cyclomaltodextrin-Glucanotransferase CGT (EC 2.4.1.19) aus *Bacillus macerans* CGT 5 h bei 37 °C inkubiert. Die so erhaltenen modifizierten Cello-Oligosaccharide werden nach Hitzedenaturierung des Enzyms (5 min bei 100 °C) durch Ultrafiltration aufgereinigt.

### Variante B)

Es wird ebenso verfahren wie bei der Variante A), wobei jedoch bei 50 °C statt bei 37 °C inkubiert wird.

### Variante C)

Es wird ebenso verfahren wie bei der Variante A), wobei jedoch bei 10 °C statt bei 37 °C inkubiert wird.

### Beispiel 5

Herstellung eines erfindungsgemäß modifizierten Kohlenhydrates b) aus einem unverdaubaren Grundkörper und mindestens einem daran angekoppelten glucosehaltigen Kohlenhydratrest.

### Variante A)

50 g Cello-Oligosaccharide (DP2 bis DP 10) und 15 g Maltodextrine in 500 ml 20 mM Acetatpuffer pH 5,2 werden mit 30000 U Cyclomaltodextrin-Glucanotransferase CGT (EC 2.4.1.19) aus *Bacillus mecerans* CGT 5 h bei 37 °C inkubiert. Die so erhaltenen modifizierten Cello-Oligosaccharide werden nach Hitzedenaturierung des Enzyms (5 min bei 100 °C) durch Ultrafiltration aufgereinigt.

### Variante B)

Es wird ebenso verfahren wie bei der Variante A), wobei jedoch bei 50 °C statt bei 37 °C inkubiert wird.

### Variante C)

Es wird ebenso verfahren wie bei der Variante A), wobei jedoch bei 10 °C statt bei 37 °C inkubiert wird.

### Beispiel 6

Herstellung eines erfindungsgemäß modifizierten Kohlenhydrates b) aus einem unverdaubaren Grundkörper und mindestens einem daran angekoppelten glucosehaltigen Kohlenhydratrest.

### Variante A)

180 g Cellobiose (DP2) und 80 g Maltodextrine in 500 ml 20 mM Acetatpuffer pH 5,2 werden mit 100 000 U Cyclomaltodextrin-Glucanotransferase CGT (EC 2.4.1.19) aus Bacillus mecerans CGT 5 h bei 37 °C inkubiert. Die so erhaltene modifizierte Cellobiose (etwa 85 g) wird nach Hitzedenaturierung des Enzyms (5 min bei 100 °C) durch Ultrafiltration aufgereinigt.

### Variante B)

Es wird ebenso verfahren wie bei der Variante A), wobei jedoch bei 50 °C statt bei 37 °C inkubiert wird.

### Variante C)

Es wird ebenso verfahren wie bei der Variante A), wobei jedoch bei 10 °C statt bei 37 °C inkubiert wird.

Das folgende Diagramm zeigt die Verdauung bzw. den Verdau der Kohlenhydratmischungen der Beispiele 1A und 6.

Die Freisetzung von Glucose aus diesen erfindungsgemäßen Kohlenhydratmischungen ist - ermittelt in einem in-vitro Verdausystem auf Pankreatinbasis - um mindestens 10 % pro Zeiteinheit reduziert, verglichen mit einer Kohlenhydratmischung, die die gleiche Gewichtsmenge an nicht modifizierten Kohlenhydraten enthält. Die Glucose wird beispielsweise enzymatisch nachgewiesen, etwa mittels des Testkits von Boehringer (Best. Nr. 139106).

## Patentansprüche

1. Verwendung eines modifizierten Kohlenhydrats a) oder b), das durch enzymatische Kopplung bzw. Verknüpfung eines üblichen, zu Ernährungszwecken eingesetzten Kohlenhydrates mit einem weiteren Kohlenhydratrest erhalten wurde, oder einer Mischung aus mehreren derartigen Kohlenhydraten zur Herstellung eines Mittels in Form eines Diabetikernahrungsmittels oder eines Pharmazeutikums für einen Diabetiker, wobei das modifizierte Kohlenhydrat a) einen Grundkörper aus einem verdaubaren, glucosehaltigen Kohlenhydrat in Form eines verdaubaren Glucanes, bei dem es sich um Maltodextrine handelt, aufweist, wobei an diesen Grundkörper mindestens ein Glucoserest und/oder ein anderer Kohlenhydratrest gekoppelt und somit damit verbunden wurde, und es sich bei den modifizierten Kohlenhydrat a) um Maltodextrine handelt,
i) die mittels einer Transglucosidase unter Ausbildung von glycosidischen Bindungen mit Glucose in α 1→2- , α 1→3- , α 1→6-, oder α 1→4-Stellung in einer Transglycosylierungsreaktion derivatisiert wurden,
ii) die mittels β-Galactosidase unter Ausbildung von glycosidischen Bindungen mit Galactose in β 1->3- , β 1->4-oder 1->6-Stellung derivatisiert wurden und/oder
iii) an deren freie Hydroxylgruppen am C2-, C3- oder C4-Kohlenstoffatom mittels Cyclomaltodextrin-Glucanotransferase CGT (EC 2.4.1.19) aus *Bacillus mecerans* CGT Glucanreste aus Amylose oder Amylopektin (aus Stärke) übertragen wurden.
und wobei das modifizierte Kohlenhydrat b) einen Grundkörper aus einem unverdaubaren Kohlenhydrat in Form eines nicht verdaubaren Speicherkohlenhydrates, Gerüstkohlenhydrates oder niedermolekularen Bestandteiles davon aufweist, wobei es sich bei dem nicht-verdaubaren Grundkörper um Fructane, β-Glucane, Cellulose, Pektine, Galacturonane, Galactane, Galactomannane, β-Galacto-Oligosaccharide, α-Galacto-Oligosaccharide, Fucoidane, Mannane, Xylane, Xyloglucane, Laminarin, Chitine, Chitosane, Hyaluronsäuren, Chondroitine, Proteoglycane, Glucurono-Oligosaccharide, Arabinane, Arabinoxylane, Arabinogalactane, Rhamno-Oligosaccharide, Xanthane, Alginate, Agar, Carageenane, Hemicellulosen, Pflanzengummi, enzymatisch hergestellte Kohlenhydrate, bakterielle Kohlenhydrate, N-Glycoprotein-Oligosaccharide, O-Glycoprotein-Oligosaccharide und Glycolipid-Oligosaccharide handelt und wobei an diesen Grundkörper mindestens ein Glucoserest und/oder ein glucosehaltigen Kohlenhydratrest gekoppelt und somit damit verbunden wurde

2. Verwendung nach Anspruch 1,
**dadurch gekennzeichnet,**
**daß** als Transglucosidase Dextransucrase aus *Leuconostoc mesenteroides* (EC 2.4.1.24) und Saccharose als Quelle für die zu koppelnde Glucose, insbesondere im Überschuß, eingesetzt werden.

3. Verwendung nach Anspruch 1,
**dadurch gekennzeichnet,**
**daß** Lactose (Gal β 1→4 Glc) und/oder Melibiose (Gal α 1→6 Glc) als Quelle für die zu koppelnde Galactose, insbesondere im Überschuß, eingesetzt werden.

4. Verwendung nach Anspruch 1,
**dadurch gekennzeichnet,**
**daß** es sich bei dem Kohlenhydrat b) um Fructane enthält, die mittels einer Transglucosidase unter Ausbildung von glycosidischen Bindungen mit Glucosen in α 1→2- , α 1→3- , α 1→6-, oder α 1→4-Stellung in einer Transglycosylierungsreaktion derivatisiert wurden.

5. Verwendung nach Anspruch 4,
**dadurch gekennzeichnet,**
**daß** als Transglucosidase aus *Aspergillus Niger* (EC 2.4.1.24) und Maltose als zu koppelnde Glucose, insbesondere im Überschuß, eingesetzt werden.

6. Verwendung nach Anspruch 1,
**dadurch gekennzeichnet,**
**daß** es sich bei dem modifizierten Kohlenhydrat b) um Fructane handelt, an deren freie Hydroxylgruppen am C2-, C3- oder C4-Kohlenstoffatom mittels Cyclomaltodextrin-Glucanotransferase CGT (EC 2.4.1.19) aus *Bacillus mecerans* CGT Glucanreste aus Amylose oder Amylopektin (aus Stärke) übertragen wurden.

## Claims

1. Use of a modified carbohydrate a) or b) obtained by coupling or linking, respectively, a usual carbohydrate used for nutritional purposes with a further carbohydrate residue, or a mixture of several of such carbohydrates for preparing a composition in the form of a food for diabetics or of a pharmaceutical for diabetics,
whereby the modified carbohydrate a) comprises a base body from a digestible glucose-containing carbohydrate in the form of a digestible glucan which is a maltodextrin, at least one glucose residue and/or another carbohydrate residue having been coupled and hence linked with said base body, and
whereby the modified carbohydrates a) are maltodextrins
i) which were derivatized in a transglycosilation reaction by means of a transglucosidase under formation of glycosidic bonds with glucose in the α 1→2 position, α 1→3 position, α 1→6 position or α 1→4 position,
ii) which were derivatized by means of β-galactosidase under formation of glycosidic bonds with galactose in the β1→3 position, β 1→4 position or the 1→6 position, and/or
iii) to the free hydroxyl groups of which at the C2, C3 or C4 carbon atom, glucan residues from amylose or amylopectin (from starch) were transferred by means of cyclomaltodextrin glucanotransferase CGT (EC 2.4.1.19) from *Bacillus macerans* CGT,
and whereby the modified carbohydrate b) comprises a base body from an indigestible carbohydrate in the form of a non-digestible reserve carbohydrate, skeletal carbohydrate or a low-molecular component thereof whereby the non-digestible base body concerning the carbohydrate b) is fructans, β-glucans, cellulose, pectins, galacturonans, galactans, galactomannans, β-galacto-oligosaccharides, α-galacto-oligosaccharides, fucoidans, mannans, xylans, xyloglucans, laminarin, chitins, chitosans, hyaluronic acids, chondroitins, proteoglycans, glucurono-oligosaccharides, arabinans, arabinoxylans, arabinogalactans, rhamno-oligosaccharides, xanthans, alginates, agar, carragheenans, hemicelluloses, natural gum, enzymatically produced carbohydrates, bacterial carbohydrates, N-glycoprotein-oligosaccharides, O-glycoprotein-oligosaccharides and glycolipid-oligosaccharides, at least one glucose residue and/or a glucose-containing carbohydrate residue having been coupled and hence linked with said base body

2. Use according to claim 1,
**characterized in**
**that**, as transglucosidase, dextransucrase from *Leuconostoc mesenteroides* (EC 2.4.1.24) and saccharose are used as the source, in particular in excess, for the glucose to be coupled.

3. Use according to claim 1,
**characterized in**
**that** lactose (Gal β 1→4 Glc) and/or melibiose (Gal α 1→6 Glc) are used, in particular in excess, as the source for the galactose to be coupled.

4. Use according to claim 1,
**characterized in**
**that**, as the modified carbohydrate b), it contains fructans that were derivatized in a transglycosilation reaction by means of a transglucosidase under formation of glycosidic bonds with glucoses in the α 1→2 position, α 1→3 position, α 1→6 position or α 1→4 position.

5. Use according to claim 4,
**characterized in**
**that**, as transglucosidase from *Aspergillus Niger* (EC 2.4.1.24) and maltose as the glucose to be coupled, in particular in excess, are used

6. Use according to claim 1,
**characterized in**
**that**, as the modified carbohydrate b), it contains fructans, to the free hydroxyl groups of which at the C2, C3 or C4 carbon atom, glucan residues from amylose or amylopectin (from starch) were transferred by means of cyclomaltodextrin glucanotransferase CGT (EC 2.4.1.19) from *Bacillus macerans* CGT.

## Revendications

1. Utilisation d'un glucide modifié a) ou b), qui est obtenu par couplage ou liaison enzymatique d'un glucide courant, utilisé à des fins alimentaires, à un autre résidu glucidique, ou d'un mélange de plusieurs glucides de ce type pour la fabrication d'une substance sous la forme d'un aliment pour diabétiques ou d'un produit pharmaceutique pour diabétiques,
dans laquelle le glucide modifié a) présente un squelette d'un glucide contenant du glucose, digestible, sous la forme d'un glucane digestible, qui est une maltodextrine,
dans laquelle au moins un résidu de glucose et/ou un autre résidu glucidique a été couplé et donc lié à ce squelette, et le glucide modifié a) est une maltodextrine,
i) qui a été dérivée en utilisant une transglucosidase, par la formation de liaisons glycosidiques avec le glucose en position α 1->2, α 1->3, α 1->6 ou α 1->4 dans une réaction de transglycosylation,
ii) qui a été dérivée en utilisant une β-galactosidase, par la formation de liaisons glycosidiques avec le galactose en position β 1->3, β 1->4 ou β 1->6,
et/ou
iii) sur les groupes hydroxyle libres de laquelle des résidus de glucane issus d'amylose ou d'amylopectine (d'amidon) ont été transférés sur l'atome C2, C3 ou C4, en utilisant la cyclomaltodextrine-glucanotransférase CGT (EC 2.4.1.19) provenant de *Bacillus macerans* CGT,
et dans laquelle le glucide modifié b) présente un squelette d'un glucide non digestible sous la forme d'un glucide de réserve non digestible, d'un squelette glucidique non digestible ou d'un composant à faible masse moléculaire de celui-ci, le squelette non digestible étant choisi parmi les fructanes, les β-glucanes, la cellulose, les pectines, les galacturonanes, les galactanes, les galactomannanes, les β-galacto-oligosaccharides, les α-galacto-oligosaccharides, les fucoïdanes, les mannanes, les xylanes, les xyloglucanes, la laminarine, les chitines, les chitosanes, les acides hyaluroniques, les chondroïtines, les protéoglycanes, les glucurono-oligosaccharides, les arabinanes, les arabinoxylanes, les arabinogalactanes, les rhamno-oligosaccharides, les xanthanes, les alginates, les géloses, les carraghénanes, les hémicelluloses, les gommes végétales, les glucides produits par voie enzymatique, les glucides produits par voie bactérienne, les N-glycoprotéine-oligosaccharides, les O-glycoprotéine-oligosaccharides et les glycolipide-oligosaccharides, au moins un résidu de glucose et/ou un résidu glucidique contenant du glucose étant couplé et donc lié à ce squelette.

2. Utilisation selon la revendication 1,
**caractérisée en ce que**
l'on met en oeuvre la dextrane-sucrase provenant de *Leuconostoc mesenteroides* (EC 2.4.1.24) en tant que transglucosidase et le saccharose en tant que source de glucose à coupler, en particulier en excès.

3. Utilisation selon la revendication 1,
**caractérisée en ce que**
l'on met en oeuvre le lactose (Gal β 1->4 Glc) et/ou le mélibiose (Gal α 1->6 Gic) en tant que source de galactose à coupler, en particulier en excès.

4. Utilisation selon la revendication 1,
**caractérisée en ce que**
le glucide b) est un fructane qui a été dérivé en utilisant une transglucosidase, par formation de liaisons glycosidiques avec des glucoses en position α 1->2, α 1->3, α 1->6 ou α 1->4 dans une réaction de transglycosylation.

5. Utilisation selon la revendication 4,
**caractérisée en ce que**
l'on met en oeuvre une transglucosidase provenant de *Aspergillus niger* (EC 2.4.1.24) et le maltose en tant que glucose à coupler, en particulier en excès.

6. Utilisation selon la revendication 1,
**caractérisée en ce que**
le glucide modifié b) est un fructane sur les groupes hydroxyle libres duquel des résidus de glucane issus d'amylose ou d'amylopectine (d'amidon) ont été transférés sur l'atome de carbone C2, C3 ou C4 en utilisant la cyclomaltodextrine-glucanotransférase CGT (EC 2.4.1.19) provenant de *Bacillus macerans* CGT.
